Europäisches Patentamt

European Patent Office

Office européen des brevets

(11) Publication number: **0 313 358**
**A1**

(12) **EUROPEAN PATENT APPLICATION**

(21) Application number: **88309873.3**

(22) Date of filing: **20.10.88**

(51) Int. Cl.⁴: **B 60 H 3/00**
**A 61 L 9/01**

(30) Priority: **23.10.87 JP 162092/87**

(43) Date of publication of application:
**26.04.89 Bulletin 89/17**

(84) Designated Contracting States: **DE FR GB IT SE**

(71) Applicant: **SANDEN CORPORATION**
**20 Kotobuki-cho**
**Isesaki-shi Gunma, 372 (JP)**

(72) Inventor: **Sakano, Riichi**
**88 Ushiro-Hanzawa Okabemachi**
**Osato-gun Saitama 369-2 (JP)**

**Isobe, Toshimi**
**2263-2 Toyoshiro-cho**
**Isesaki-shi Gunma, 372 (JP)**

(74) Representative: **Brunner, Michael John et al**
**GILL JENNINGS & EVERY 53-64 Chancery Lane**
**London WC2A 1HN (GB)**

(54) Automotive air conditioning system.

(57) A ventilating device for an automotive air conditioning system has a duct (10) in which a blower (30) and an evaporator assembly (40) are diposed. The evaporator assembly (40) includes an evaporator (41), a plastic element (44,45) surrounding the evaporator and a deodorizing member (46). This deodorizing member may include a deodorant such as Phytoncide. Alternatively, the deodorant may be impregnated in the plastic element which surrounds the evaporator (41).

Fig. 1

EP 0 313 358 A1

**Description**

## AUTOMOTIVE AIR CONDITIONING SYSTEM

This invention relates to a deodorizing device for a passenger compartment of an automobile.

To deodorize a passenger compartment of an automobile, it is known to dispose a device within the passenger compartment of the automobile and provide the device with a volatile deodorant to deodorize the passenger compartment of the automobile by vaporization of the deodorant. A device which sprays liquefied deodorant into the passenger compartment of the automobile to deodorize the passenger compartment of the automobile has also been used.

While the above-mentioned devices are capable of deodorizing the air in the passenger compartment of the automobile, the above-mentioned devices do not eliminate the offensive odor which is produced by bacteria and mold found in an evaporator assembly of an automotive air-conditioning system. Accordingly, when air blows from the evaporator into the passenger compartment during operation of the air conditioner, more particularly, at the start of an air-conditioning operation, the above-mentioned devices fail to deodorize the passenger compartment of the automobile sufficiently.

A device which sprays liquefied deodorant into a duct of a ventilating device of an automotive air-conditioning system to eliminate or minimise offensive odors is known. However, such a device only lasts a short time. Therefore, spraying must be repeated to maintain deodorization. Furthermore, such a device is expensive and must be thrown away after only one use, so that it is costly for the effect that it achieves.

It is a primary object of this invention to eliminate or minimize the offensive odor which is produced by bacteria and mold found in an evaporator assembly of an automotive air conditioning system.

It is another object of this invention to obtain long-life deodorization inexpensively.

According to the invention, a ventilating device for an automotive air conditioning system comprises a duct, a blower and an evaporator assembly, the blower and evaporator being disposed within the duct and the evaporator assembly including an evaporator and a deodorizing member. The deodorizing member may include a deodorant such as Phytoncide which may be impregnated in to a plastics element which surrounds the evaporator.

One example of an automobile air-conditioning system constructed in accordance with the present invention will now be described with reference to the accompanying drawings, in which:-

Figure 1 is a schematic block diagram of a ventilating device of an automotive air conditioning system; and,

Figure 2 is an exploded perspective illustration of an evaporator assembly of the ventilating device.

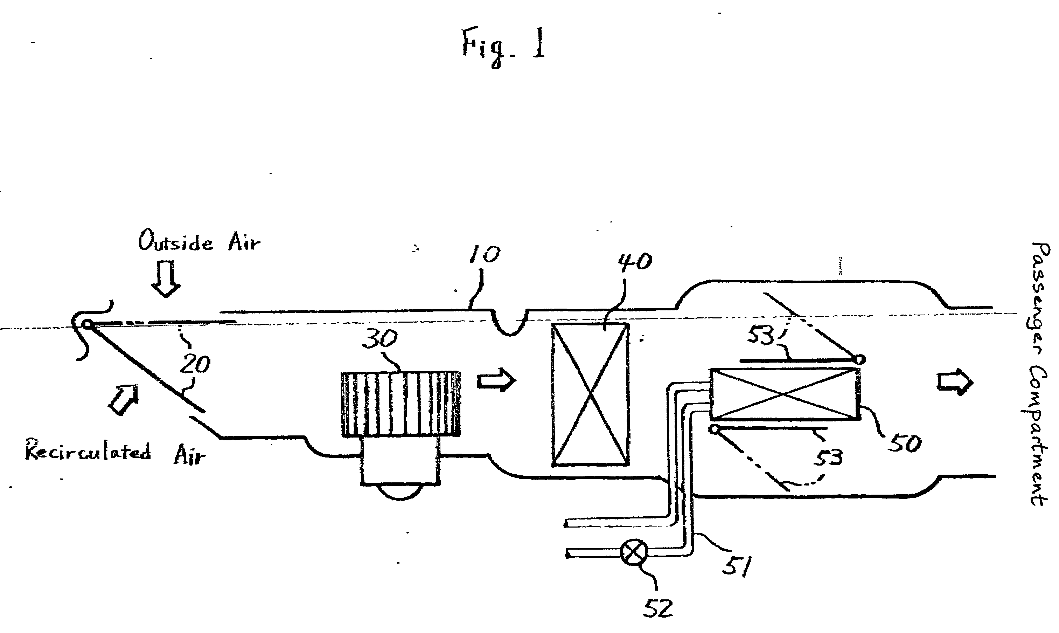

For purposes of explanation only the left side of Figure 1 will be referred to as the forward end and the right side as the rearward end.

The ventilating device comprises a duct 10, a blower 30, an evaporator assembly 40, and a heating core 50. A rearward end opening of the duct 10 opens to the passenger compartment of an automobile (not shown). A first damper 20 controls the intake of outside air and recirculated air from the passenger compartment and is disposed at a forward end opening of the duct 10. The first damper 20 is illustrated as a solid line and a double dotted line to represent the position of the damper in an outside air intake mode and a recirculated air intake mode, respectively. The blower 30 disposed within the duct 10, blows air from the first damper 20 to the passenger compartment through the duct 10. The evaporator assembly 40 is disposed within the duct 10 on the rearward side of the blower 30, and the heating core 50 is disposed within the duct 10 on the rearward side of the evaporator assembly 40. A water pipe 51 through which hot water circulates is connected to the heating core 50 and is provided with a valve 52 which controls the circulation of the hot water. Second dampers 53, disposed adjacent the heating core 50, control the inflow of air to the heating core 50. Second dampers 53 are illustrated as solid lines and double dotted lines to represent no inflow of air and maximum inflow of air, respectively.

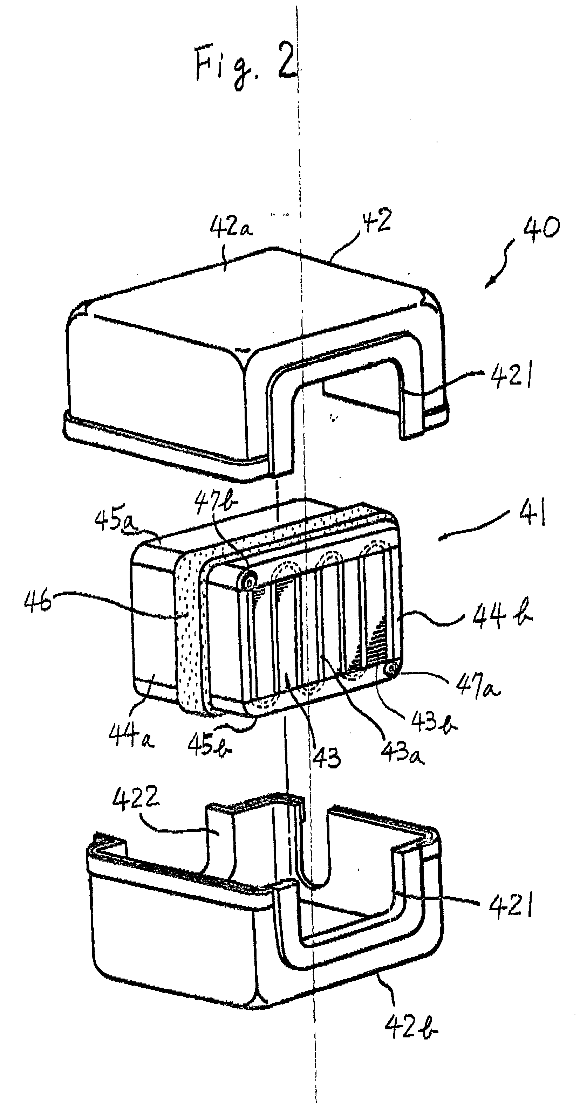

The evaporator assembly 40 shown in Figure 2 comprises an evaporator 41 and a container 42 including an open-box shaped upper container 42a and an open-box shaped lower container 42b, which are made of Polypropylene.

Upper and lower containers 42a and 42b each include a substantially rectangular base having a plurality of substantially rectangular mutually perpendicular side walls. One or more of the side walls may have a substantially U-shaped cutout formed therein. The evaporator 41 is housed within container 42 by joining together the upper and lower containers 42a, 42b with the evaporator 41 located therein. The lower container 42b receives liquid which is condensed at a surface of the evaporator 41 and drains the liquid to the outside of the automobile through a drain pipe (not shown) connected to the lower container 42b. An air intake opening 421 and an air blow-off opening 422 are formed at a forward end surface and a rearward end surface of the container 42 respectively. Openings 421 and 422 may correspond in position to the U-shaped cutouts described above.

The evaporator 41 comprises a heat exchange portion 43 and side walls 44a, 44b, which are made of aluminium alloy and which are attached at both side end surfaces of heat exchange portion 43. The heat exchange portion 43 includes a flat shaped pipe 43a which follows a vertical serpentine path, and fin members 48b which are attached to the pipe 43a by brazing. The front and rear end surfaces of the exchange portion 43 face the air intake opening 421 and the air blow-off opening 422 respectively. Covers 45a, 45b, which are made of polystyrene, are disposed at the top and bottom ends of the evaporator 41 and cover the top and bottom of evaporator 41 to protect it from damage. Evaporator inlet and outlet pipes 47a, 47b connect to the inlet and outlet portions of the flat shaped pipe 43a, respectively.

A foamed belt member 46, which is made of polyurethene, surrounds both side walls 44a, 44b and both covers 45a, 45b to effectively divide an inner space of container 42 into two separate airtight regions, a first region defining an air intake region, and the second an air blow-off region. The belt member 46 is impregnated with a deodorant, for instance, Phytoncide which is extracted from a needle-leaf tree as a deodorant and an anti-bacteria. In nature, Phytoncide protects trees from bacteria and microorganisms.

In operation, when the automotive air conditioning system works, intake air flows from the forward end opening of the duct 10 to the rearward end opening. Accordingly, intake air can flow through the evaporator 40 so that it is cooled down by exchanging heat with supercooled refrigerant in the evaporator 40. Furthermore, any offensive odor, which is produced by bacteria and mould found in the lower container 42b acting as a drain pan and fin members 43b, is eliminated or minimized by Phytoncide which is released from the foamed belt member 46 so that deodorized air blows into the passenger compartment.

In the above mentioned embodiment, foamed belt member 46 is used for a deodorizing member by impregnating the belt with a deodorant. However, the container 42 and/or covers 45a, 45b may be used as a deodorizing member by mixing deodorant during the manufacture of the container and/or covers.

Furthermore, this enables deodorization to occur over a long period of time without any intervention by a user. Preferably, the deodorizing member is designed to last for the entire life of the evaporator.

## Claims

1. An automobile air conditioning system for providing air to a passenger compartment of an automobile by means of a ventilating system comprising a duct (10) in which a blower (30) and an evaporator assembly (40) are disposed, characterised by:
deodorizing means (46) located in the duct for deodorizing the air in the ventilating system prior to it being passed to the passenger compartment.

2. A system according to claim 1, wherein the deodorizing means (46) comprises a part of the evaporator assembly (40) and includes a deodorant.

3. A system according to claim 1 or claim 2, wherein the evaporator assembly comprises an evaporator (41) and an element (46) which surrounds at least a portion of the evaporator; the deodorizing means (46) including a deodorant and forming at least a portion (46) of the element.

4. A system according to claim 1, wherein the evaporator assembly comprises container means (42a,42b) for housing an evaporator (41) and the deodorizing means comprises at least a portion of the container means and a deodorant.

5. A system according to claim 1, wherein the evaporator assembly comprises an evaporator (41) and a belt (46) surrounding the evaporator, the deodorizing means comprising at least a portion of the belt (46) and a deodorant.

6. A system according to claim 1, wherein the evaporator assembly comprises an evaporator (41), cover means (45a,454b) for covering top and bottom portions of the evaporator, the deodorizing mean comprising at least a portion of the cover means and a deodorant.

7. A system according to claim 3, wherein the element comprises a cover means (45a,45b) for covering top and bottom portions of the evaporator (41), container means for housing the evaporator and a belt (46) surrounding the evaporator and the cover means.

8. A system according to claim 3 or claim 7, wherein the element retains the deodorant.

9. A system according to claim 3 or claim 7, wherein the deodorant is impregnated into at least a portion of the element.

10. A system according to any of claims 2 to 9, wherein the deodorant is Phytoncide.

11. A system according to claim 4, wherein the container means is made of polypropylene.

12. A system according to claim 6, wherein the cover means is made of polystyrene.

13. A system according to claim 5, wherein the belt is made of polystyrene.

Fig. 1

# Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 097 287 (J.FR. BEHR) <br> * Whole document * | 1 | B 60 H 3/00 <br> A 61 L 9/01 |
| A | | 3,4,8,9 | |
| X | PATENT ABSTRACTS OF JAPAN, vol. 10, no. 159 (M-486)[2215], 7th June 1986; & JP-A-61 12 421 (NISSAN JIDOSHA K.K.) 20-01-1986 <br> * Abstract * | 1 | |
| X | PATENT ABSTRACTS OF JAPAN, vol. 7, no. 112 (M-215)[1257], 17th May 1983; & JP-A-58 33 511 (NIPPON DENSO K.K.) 26-02-1983 <br> * Abstract * | 1 | |
| P,X | PATENT ABSTRACTS OF JAPAN, vol. 11, no. 356 (M-644)[2803], 20th November 1987; & JP-A-62 134 318 (NISSAN MOTOR CO., LTD) 17-06-1987 <br> * Abstract * | 1 | |
| P,Y | PATENT ABSTRACTS OF JAPAN, vol. 11, no 393 (M-654)[2840], 23rd December 1987; & JP-A-62 160 913 (DIESEL KIKI CO., LTD) 16-07-1987 <br> * Abstract * | 1-4,6,8 ,9 | TECHNICAL FIELDS SEARCHED (Int. Cl.4) <br><br> B 60 H <br> A 61 G <br> F 25 D <br> A 61 L |
| Y | PATENT ABSTRACTS OF JAPAN, vol. 11, no. 272 (M-622)[2719], 4th September 1987; & JP-A-62 74 707 (NIPPON RADIATOR CO., LTD) 06-04-1987 <br> * Abstract * | 1-4,6,8 ,9 | |
| A | IDEM <br> ---      -/- | 5,7 | |

The present search report has been drawn up for all claims

| Place of search | Date of completion of the search | Examiner |
|---|---|---|
| THE HAGUE | 18-01-1989 | CZAJKOWSKI A.R. |

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | PATENT ABSTRACTS OF JAPAN, vol. 11, no. 128 (M-583)[2575], 22nd April 1987; & JP-A-61 268 934 (TOYODA GOSEI CO., LTD) 28-11-1986 --- | 10 | |
| A | EP-A-0 080 171 (HITACHI LTD) * Abstract; figure 1 * ----- | 11 | |

TECHNICAL FIELDS
SEARCHED (Int. Cl.4)

The present search report has been drawn up for all claims

| Place of search | Date of completion of the search | Examiner |
|---|---|---|
| THE HAGUE | 18-01-1989 | CZAJKOWSKI A.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)